(19) Europäisches Patentamt
European Patent Office
Office européen des brevets

(11) Veröffentlichungsnummer: **0 098 445**
**A2**

(12) # EUROPÄISCHE PATENTANMELDUNG

(21) Anmeldenummer: **83106021.5**

(22) Anmeldetag: **21.06.83**

(51) Int. Cl.³: **C 07 D 277/58**
// C09B29/042

(30) Priorität: **08.07.82 DE 3225472**

(43) Veröffentlichungstag der Anmeldung: **18.01.84**
**Patentblatt 84/3**

(84) Benannte Vertragsstaaten: **CH DE FR GB IT LI**

(71) Anmelder: **CASSELLA Aktiengesellschaft, Hanauer Landstrasse 526, D-6000 Frankfurt am Main 61 (DE)**

(72) Erfinder: **Schuster, Karl-Heinz, Dr., Adalbert-Stifter-Strasse 35, D-6457 Maintal 2 (DE)**
Erfinder: **Müller, Rolf, Dr., Dornbachweg 3, D-6367 Karben 4 (DE)**
Erfinder: **Tappe, Horst, Dr., Ringstrasse 9, D-6057 Dietzenbach (DE)**
Erfinder: **Wille, Herbert, Dr., Hünfelder Strasse 16, D-6000 Frankfurt am Main 61 (DE)**

(74) Vertreter: **Urbach, Hans-Georg, Dr. et al, Hanauer Landstrasse 526, D-6000 Frankfurt am Main 61 (DE)**

(54) **Verfahren zur Herstellung von 2-Amino-5-nitro-thiazol.**

(57) Bei dem Verfahren zur Herstellung von 2-Amino-5-nitro-thiazol wird eine schwefelsaure Lösung von 2-Amino-5-nitro-thiazol, die mindestens 6 Gew.-% 2-Amino-5-nitro-thiazol in einer mehr als 80gewichtsprozentigen Schwefelsäure enthält, auf eine Schwefelsäurekonzentration von weniger als 80 Gew.-% verdünnt und der dabei ausfallende Niederschlag abgetrennt und hydrolytisch zu 2-Amino-5-nitro-thiazol zersetzt.

EP 0 098 445 A2

Ref. 3256 0098445
Dr.Lu/lro/St

# Verfahren zur Herstellung von 2-Amino-5-nitro-thiazol

Die Erfindung betrifft ein Verfahren zur Herstellung von 2-Amino-5-nitro-thiazol.

2-Amino-5-nitro-thiazol wird u.a. als Tierarzneimittel, z.B. bei der Behandlung der Entero-Hepatitis bei Truthühnern, ferner als Zwischenprodukt zur Herstellung von pharmakologisch wirksamen Substanzen und als Zwischenprodukt bei der Herstellung von Azofarbstoffen verwendet. Die direkte Nitrierung von 2-Amino-thiazol ist wegen der Gefahr einer stürmischen exothermen Reaktion nicht ungefährlich und liefert zudem auch nicht befriedigende Qualitäten und Ausbeuten.

Schwefelsaure Lösungen von 2-Amino-5-nitro-thiazol können einfach, wirtschaftlich und gefahrlos nach dem in der DE- C-2105156 beschriebenen Verfahren dadurch hergestellt werden, daß 2-Aminothiazol-bisulfit bei maximal 30°C in mindestens die dreifache Gewichtsmenge mindestens 90gewichtsprozentige Schwefelsäure eingetragen und bei maximal 30°C unter üblichen Bedingungen nitriert wird. Die so erhaltenen schwefelsauren Lösungen des 2-Amino-5-nitro-thiazols werden in der Regel direkt weiterverarbeitet, z.B. zur Herstellung von Farbstoffen diazotiert. Falls eine Isolierung des 2-Amino-5-nitro-thiazols gewünscht wird, müssen die schwefelsauren Lösungen des 2-Amino-5-nitro-thiazols weitgehend neutralisiert und das abgeschiedene 2-Amino-5-nitro-thiazol abgetrennt werden. Die Neutralisation der großen Schwefelsäuremengen ist jedoch aufwendig und mit dem Anfall unerwünschter Sulfate verbunden und deshalb ökologisch ungünstig.

Es wurde nun überraschenderweise gefunden, daß 2-Amino-5-nitro-thiazol leicht aus derartigen schwefelsauren Lösungen isoliert werden kann, ohne daß große Schwefelsäuremengen neutralisiert werden müssen, falls die schwefel-

BAD ORIGINAL

saure Lösung 6 Gewichtsprozent oder mehr 2-Amino-5-nitro-thiazol enthält. Das erfindungsgemäße Verfahren ist dadurch gekennzeichnet, daß eine mindestens 6gewichtsprozentige Lösung von 2-Amino-5-nitro-thiazol in einer mehr als 80gewichtsprozentigen Schwefelsäure auf eine Schwefelsäurekonzentration von weniger als 80 Gewichtsprozent verdünnt wird, der dabei ausfallende Niederschlag abgetrennt und aus ihm durch Hydrolyse 2-Amino-5-nitro-thiazol gewonnen wird.

Die Gehalts- bzw. die Konzentrationsangaben für die Schwefelsäure beziehen sich im Rahmen der vorliegenden Erfindung nur auf die Mengen der Komponenten Wasser und $H_2SO_4$ in den Lösungen bzw. Suspensionen.

Die Herstellung von schwefelsauren Lösungen des 2-Amino-5-nitro-thiazol , die als Ausgangsprodukt für das erfindungsgemäße Verfahren geeignet sind, kann z.B. nach dem Verfahren der DE-C- 2105156 erfolgen. Die nach diesem Verfahren herstellbaren schwefelsauren Lösungen enthalten normalerweise 6 bis 11 Gewichtsprozent 2-Amino-5-nitro-thiazol in einer 85- bis 98gewichtsprozentigen Schwefelsäure. Derartige Lösungen, die selbstverständlich auch nach einem anderen geeigneten Verfahren hergestellt worden sein können, werden erfindungsgemäß auf eine Schwefelsäurekonzentration von weniger als 80 Gewichtsprozent, insbesondere eine Schwefelsäurekonzentration von 40 bis 80 Gewichtsprozent, vorzugsweise eine Schwefelsäurekonzentration von 50 bis 70 Gewichtsprozent, verdünnt. Die Verdünnung geschieht in an sich bekannter Weise, z.B. durch Zugabe von Wasser, Eis oder verdünnter Schwefelsäure zweckmäßigerweise unter Rühren und evtl. Kühlen. Zweckmäßigerweise wird die Temperatur bei dem Verdünnungsvorgang unter 30°C gehalten. Bei diesem Verdünnungsvorgang scheidet sich eine schwerlösliche 2-Amino-5-nitro-thiazol enthaltende Verbindung ab, die nach den vorgenommenen Untersuchungen das noch nicht beschriebene 2-Amino-5-nitro-thiazol-hydrogensulfat dar-

BAD ORIGINAL

stellt. Zur Vervollständigung des Abscheidevorgangs wird nach der Verdünnung zweckmäßigerweise nachgerührt oder der Ansatz einige Zeit sich selbst überlassen. Dann wird der ausgeschiedene Niederschlag abgetrennt, was z.B. durch Abfiltrieren, Absaugen oder Abschleudern erfolgen kann. Der abgetrennte Niederschlag wird hydrolytisch zersetzt und das 2-Amino-5-nitro-thiazol auf übliche Weise gewonnen.

Die hydrolytische Zersetzung des 2-Amino-5-nitro-thiazol-hydrogensulfats wird am einfachsten durch Eintragen des Niederschlags in Wasser, zweckmäßigerweise unter Rühren, vorgenommen. Bei der Hydrolyse wird die Temperatur des vorgelegten Wassers durch Kühlung und/oder Verwendung von Eiswasser möglichst niedrig, insbesondere unter 30°C und vorzugsweise zwischen 0 und 20°C, gehalten. Es ist zweckmäßig, mindestens die gleiche, vorzugsw. mindestens die 1,4-fache, Gewichtsmenge Wasser vorzulegen. Mehr als die 8-fache Gewichtsmenge Wasser vorzulegen, bringt keine Vorteile. Vorzugsweise wird die 1,4- bis 3-fache Gewichtsmenge Wasser vorgelegt. Nach der Eintragung des 2-Amino-5-nitro-thiazol-hydrogensulfats in Wasser kann das abgeschiedene 2-Amino-5-nitro-thiazol in an sich bekannter Weise abgetrennt, z.B. abfiltriert, abgesaugt oder abgeschleudert, und getrocknet werden. Die hydrolytische Zersetzung des 2-Amino-5-nitro-thiazol-hydrogensulfats kann selbstverständlich nicht nur mit Wasser sondern auch mit wäßrigen Lösungen, wie z.B. wäßrigen Säuren oder Laugen, wie z.B. verdünnter Schwefel- oder Salzsäure, Natronlauge oder Ammoniakwasser, durchgeführt werden.

Die bei dem erfindungsgemäßen Verfahren nach der Abtrennung des ausgefallenen 2-Amino-5-nitro-thiazol-hydrogensulfats anfallenden verdünnten schwefelsauren Lösungen enthalten noch ca. 4 Gewichtsprozent 2-Amino-5-nitro-thiazol und werden zweckmäßigerweise in dieser Form weiterverwendet, z.B. zur Herstellung von Farbstoffen, wozu

BAD ORIGINAL

die Lösungen zunächst in an sich bekannter Weise diazotiert und anschließend mit einer geeigneten Kupplungskomponente gekuppelt werden. Farbstoffe, die sich so herstellen lassen, sind z.B. beschrieben in DE-C-10 19 415, EP-A-30 028, DE-A1-26 33 910.

**Es ist zweckmäßig, als Ausgangsprodukt für das erfindungsgemäße Verfahren eine schwefelsaure Lösung mit einem möglichst hohen Gehalt an 2-Amino-5-nitro-thiazol einzusetzen. Schwefelsaure Lösungen mit einem wesentlich höheren Gehalt als 11 Gew.% 2-Amino-5-nitro-thiazol lassen sich nach dem Verfahren der DE-C- 2105156 nicht herstellen. Die Herstellung derartiger Lösungen gelingt jedoch in einfacher** Weise, wenn bei diesem Verfahren, bei dem 2-Amino-thiazol-bisulfit in mindestens die 3-fache Gewichtsmenge mindestens 90 gewichtsprozentiger Schwefelsäure eingetragen und unter üblichen Bedingungen nitriert wird, nach der Beendigung der Nitrierung die Schwefelsäurekonzentration der Lösung wieder auf mindestens 90 Gewichtsprozent eingestellt, von neuem 2-Amino-thiazolbisulfit eingetragen und nochmals unter üblichen Bedingungen nitriert wird. Auf diese Weise lassen sich Lösungen mit einer Schwefelsäurekonzentration von 88 bis 97 Gewichtsprozent und einem Gehalt an 2-Amino-5-nitro-thiazol von 12 bis 21 Gewichtsprozent herstellen. Durch mehrfache Wiederholung kann die Konzentration an 2-Amino-5-nitro-thiazol weiter erhöht werden. Bei insgesamt dreimaliger Nitrierung erhält man Konzentrationen an 2-Amino-5-nitro-thiazol von 15 bis 25 Gewichtsprozent. Bei insgesamt 6- bis 10-maliger Nitrierung erhält man Konzentrationen an 2-Amino-5-nitro-thiazol von 25 bis 33 Gewichtsprozent. Aus Sicherheitsgründen ist es dabei zweckmäßig, wenn die Eintragung des 2-Amino-thiazol-bisulfits und die Nitrierung bei Temperaturen von maximal 30°C durchgeführt werden.

BAD ORIGINAL

In den nachfolgenden Beispielen sind Konzentrationsangaben in Gewichtsprozenten gemacht.

Beispiel 1

1100 g 100%ige Schwefelsäure werden auf 10°C gekühlt. Dann werden 182 g 2-Aminothiazol-bisulfit (100%ig) eingetragen und ein Teil des entstandenen Schwefeldioxids durch Absaugen oder Ausblasen entfernt. Danach werden 70 g handelsüblicher Mischsäure 90 : 10 (Gemisch aus konzentrierter Salpeter- und Schwefelsäure im Gewichtsverhältnis 90 : 10) bei 25 - 30°C zugetropft. Den exakten Endpunkt der Nitrierung zeigt eine eingehängte Platinelektrode gegen die Kalomelelektrode durch einen Potentialanstieg von etwa 750 mV auf 1000 mV an. Dann werden 221,5 g Oleum (65 % $SO_3$) zugetropft. Anschließend werden erneut 182 g 2-Aminothiazol-bisulfit eingetragen, und dann wird wie oben beschrieben nitriert. Der Vorgang wird noch einmal wiederholt. Man erhält ca. 2100 g einer schwefelsauren Lösung, die nach der spektroskopischen Gehaltsbestimmung 20,3 Gew.% 2-Amino-5-nitro-thiazol enthält, entsprechend 98 % d. Th.

Durch Zusatz von 720 g Eis wird die Schwefelsäure verdünnt. Es fällt ein Niederschlag von 2-Amino-5-nitro-thiazol-hydrogensulfat aus. Es wird 1 Stunde nachgerührt und dann der Niederschlag abgesaugt. Man erhält ca. 680 g Niederschlag sowie 2140 g Filtrat, das in einer 62,5%igen Schwefelsäure 4 Gew.% 2-Amino-5-nitro-thiazol (bestimmt durch UV-Spektroskopie) enthält. Der erhaltene Niederschlag wird unter Kühlen im Eisbad auf 1500 g Eiswasser eingetragen, der durch Zersetzung entstandene Niederschlag abgesaugt und nachgewaschen. Das 2-Amino-5-nitro-thiazol wird getrocknet. Man erhält ca. 320 g chromatographisch reines Produkt vom Schmelzpunkt 200 - 202°C (Zers.).

Falls erwünscht, kann das hierbei erhaltene Filtrat zum Verdünnen des nächsten Ansatzes benutzt werden.

Daten des als Zwischenstufe erhaltenen 2-Amino-5-nitro-thiazol-hydrogensulfats:

Analyse: $C_3H_5N_3O_6S_2$

| | | | | | | |
|---|---|---|---|---|---|---|
| Ber.: | C 14,8 | H 2,1 | O 39,5 | N 17,3 | S 26,4 | $SO_4^{--}$ 39,5 |
| Gef.: | C 14,8 | H 2,0 | O 39,9 | N 16,7 | S 26,4 | $SO_4^{--}$ 39,9 |

$\lambda$max. : 375 nm (Methanol)
: 385 nm ($H_2O$)

Schmelzpunkt: 167 - 169°C

Die angegebene Struktur wurde durch IR- und NMR-Spektroskopie bestätigt.

Beispiel 2

Die nach Beispiel 1 erhaltenen 2140 g Filtrat, enthaltend 4 Gew.% 2-Amino-5-nitro-thiazol, werden in üblicher Weise diazotiert und auf N-Phenethyl-N-cyanethylanilin gekuppelt. Es wurde so der Farbstoff der Formel

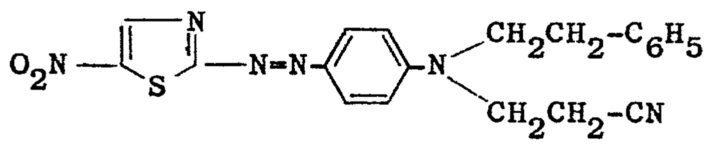

erhalten.

Beispiel 3

Es wurde wie im Beispiel 1, nur bei Nitriertemperaturen von 15 bis 20°C, gearbeitet. Man erhält 2-Amino-5-nitro-thiazol in der gleichen Menge und Reinheit.

Beispiel 4

Bei der Wiederholung von Beispiel 1 wurde die Mischsäure 90 : 10 durch andere übliche Nitrierungsagentien, z.B. Salpetersäure, Mischsäure 50 : 50 oder Lösungen von Nitraten in Schwefelsäure ersetzt. Man erhält 2-Amino-5-nitro-thiazol in praktisch identischer Menge und Reinheit.

Beispiel 5:

Bei der Wiederholung von Beispiel 1 werden statt 221,5 g Oleum 246g Oleum (65% $SO_3$) zugesetzt. Man erhält 2-Amino-5-nitro-thiazol in der gleichen Menge und Reinheit.

Beispiel 6

Zu 2100 g der nach Beispiel 1 erhaltenen schwefelsauren Lösung von 2-Amino-5-nitro-thiazol werden 246 g Oleum (65 % $SO_3$) zugetropft. Anschließend werden erneut 182 g 2-Aminothiazol-bisulfit eingetragen und dann wird wie im Beispiel 1 beschrieben nitriert. Dieser Schritt wird noch zweimal wiederholt.

Man erhält ca. 3400 g einer schwefelsauren Lösung, die nach der spektroskopischen Gehaltsbestimmung 24,8 Gew.% 2-Amino-5-nitro-thiazol enthält, entsprechend 97 % d.Th.

Durch Zusatz von 1440 g Eis wird die Schwefelsäure verdünnt. Es fällt 2-Amino-5-nitro-thiazol-hydrogensulfat aus. Es wird 1 Stunde nachgerührt und dann abgesaugt. Man erhält ca.1360 g Niederschlag sowie 3480 g Filtrat, enthaltend 3,9 Gew.% 2-Amino-5-nitro-thiazol (bestimmt durch UV-Spektroskopie) in einer ca. 63 gew.%igen Schwefelsäure. Der Niederschlag wird wie im Beispiel 1 beschrieben hydrolysiert. Man erhält ca. 660 g chromatographisch reines 2-Amino-5-nitro-thiazol vom Schmelzpunkt 200 - 202°C (Zers.) Das Filtrat wird wie im Beispiel 2 beschrieben weiter umgesetzt.

Beispiel 7

Die nach Beispiel 1 der DE-C-21 05 156 erhaltene schwefelsaure Lösung wird mit 950 g Eis verdünnt, wobei ein Niederschlag von 2-Amino-5-nitro-thiazol-hydrogensulfat ausfällt. Es wird 1 Stunde nachgerührt und dann abgesaugt. Man erhält 200 g Niederschlag sowie 2800 g Filtrat, enthaltend 4 Gew.% 2-Amino-5-nitro-thiazol (bestimmt durch UV-Spektroskopie) in einer 62,5 gew.%igen Schwefelsäure.

Der Niederschlag wird mit 450 g Eiswasser zersetzt, abgesaugt und nachgewaschen. Das 2-Amino-5-nitro-thiazol wird getrocknet. Man erhält 75 g chromatographisch reines 2-Amino-5-nitro-thiazol vom Schmelzpunkt 200 - 202°C (Zers.).

Patentansprüche

1. Verfahren zur Herstellung von 2-Amino-5-nitro-thiazol, dadurch gekennzeichnet, daß eine mehr als 6gewichtsprozentige Lösung von 2-Amino-5-nitro-thiazol in einer mehr als 80gewichtsprozentigen Schwefelsäure auf eine Schwefelsäurekonzentration von weniger als 80 Gew.% verdünnt wird, der dabei ausfallende Niederschlag abgetrennt und aus ihm durch Hydrolyse 2-Amino-5-nitro-thiazol gewonnen wird.

2. Verfahren nach Anspruch 1, dadurch gekennzeichnet, daß die Schwefelsäurelösung auf eine Schwefelsäurekonzentration von 40 bis 80 Gew.%, vorzugsweise von 50 bis 70 Gew.%, verdünnt wird.

3. Verfahren nach Anspruch 1 und/oder 2, dadurch gekennzeichnet, daß eine Schwefelsäurelösung verdünnt wird, die 6 bis 33 Gew.%, vorzugsweise 15 bis 25 Gew.%, 2-Amino-5-nitro-thiazol enthält.

4. Verfahren zur Herstellung von schwefelsauren Lösungen, die mehr als 6 Gew.% 2-Amino-5-nitro-thiazol enthalten und die sich insbesondere als Ausgangsprodukt für das Verfahren nach einem oder mehreren der Ansprüche 1 bis 3 eignen, wobei 2-Amino-thiazol-bisulfit in mindestens die dreifache Gewichtsmenge mindestens 90gew.%iger Schwefelsäure eingetragen und unter üblichen Bedingungen nitriert wird, dadurch gekennzeichnet gekennzeichnet, daß nach der Beendigung der Nitrierung die Schwefelsäurekonzentration der Lösung wieder auf mindestens 90 Gew.% eingestellt, von neuem 2-Amino-thiazol-bisulfit eingetragen und nochmals unter üblichen Bedingungen nitriert wird.

5. Verfahren nach Anspruch 4, dadurch gekennzeichnet, daß es 2- bis 10-fach wiederholt wird.

6. Verfahren nach Anspruch 4 und/oder 5, dadurch gekennzeichnet, daß die Eintragung des 2-Amino-thiazol-bisulfits und die Nitrierung bei Temperaturen von maximal 30°C durchgeführt werden.

7. Verfahren nach den Ansprüchen 1 bis 3, dadurch gekennzeichnet, daß die nach Abtrennung des Niederschlags zurückbleibende Schwefelsäurelösung zur Herstellung von Farbstoffen verwendet wird.

8. 2-Amino-5-nitro-thiazol-hydrogensulfat.